(19) Europäisches Patentamt
European Patent Office
Office européen des brevets

(11) Publication number: 0 087 870 B1

(12) EUROPEAN PATENT SPECIFICATION

(45) Date of publication of patent specification: 17.04.85

(51) Int. Cl.[4]: C 07 C 51/56, C 07 C 51/12, C 07 C 53/08, C 07 C 53/12

(21) Application number: 83300662.0

(22) Date of filing: 10.02.83

# (54) Process for the production of acetic anhydride and acetic acid.

(30) Priority: 13.02.82 GB 8204284
29.04.82 GB 8212527

(43) Date of publication of application:
07.09.83 Bulletin 83/36

(45) Publication of the grant of the patent:
17.04.85 Bulletin 85/16

(84) Designated Contracting States:
BE DE FR GB IT NL

(56) References cited:
DE-A-2 450 965
DE-A-2 844 371
DE-C- 921 938
US-A-4 039 395

(73) Proprietor: BP Chemicals Limited
Belgrave House 76 Buckingham Palace Road
London, SW1W 0SU (GB)

(72) Inventor: Cooper, Jeremy Bernard BP Chemicals Limited
Belgrave House 76 Buckingham Palace Road
London SW1W 0SU (GB)

(74) Representative: Crack, Richard David et al
c/o The British Petroleum Company plc Patents Division Chertsey Road
Sunbury-on-Thames Middlesex, TW16 7LN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 087 870 B1

## Description

The present invention relates to a process for the production of acetic anhydride with or without the net co-production of acetic acid.

Acetic acid and acetic anhydride have been known as industrial chemicals for many years. Acetic anhydride constitutes the second largest end use for acetic acid and is widely employed in the production of cellulose acetate and other cellulose esters. Smaller quantities are used in the production of specialist esters, aspirin and pesticides. Acetic acid is used as a preservative and as an intermediate in the production of for example acetate esters.

Until recently acetic acid has almost exclusively been produced on an industrial scale by the oxidation of petroleum-derived fractions. Now, however, its production by the rhodium catalysed carbonylation of methanol is gaining increasing importance. On an industrial scale acetic anhydride is currently produced either by reaction of ketene and acetic acid, the ketene being obtained either by dehydration of acetic acid or by decomposition of acetone, or by oxidation of acetaldehyde which also yields acetic acid. Each of these conventional routes has well-known disadvantages which knowledge, together with the increasing attention being paid to chemical syntheses involving carbon monoxide and carbon monoxide/hydrogen mixtures, has led to investigations into the production of acetic anhydride utilising these materials.

British Patent No. 1468940 (Halcon International Inc.) describes and claims a process for the preparation of an anhydride of a monocarboxylic acid which comprises reacting a carboxylate ester satisfying the formula RCOOR or an ether satisfying the formula ROR with an acyl halide satisfying the formula RCOX, formed in situ or in a separate stage, under substantially anhydrous conditions, wherein X is iodide or bromide, the Rs may be the same or different and each R is a monovalent hydrocarbyl radical or a substituted monovalent hydrocarbon radical wherein the or each substituent is inert. The acyl halide may be produced by carbonylation of a halide satisfying the formula RX at superatmospheric pressure, R and X being as hereinbefore defined, and the carbonylation may be effected in the presence as catalyst of a Group VIII noble metal, i.e. iridium, osmium, platinum, palladium, rhodium and ruthenium, and optionally a promoter selected from elements having atomic weights greater than 5 of Groups IA, IIA, IIIA, IVB, VIB, the non-noble metals of Group VIII and the metals of the lanthanide and actinide groups of the Periodic Table, of which suitable metals are lithium, magnesium, calcium, titanium, chromium, iron, nickel and aluminium. It is stated in GB 1468940 that it is important that the carbonylation reaction should be carried out under substantially anhydrous conditions, i.e. the reactants should be essentially dry.

In GB 1 523 346 (Hoechst Aktiengesellschaft) the process is disclosed for the preparation of acetic anhydride which comprises treating methyl acetate with carbon monoxide in the presence of a metal selected from ruthenium, rhodium, palladium, osmium, iridium and platinum, hereinafter referred to as a noble metal, in free or combined form and of a halogen in free or combined form. In this specification it is stated that the reaction is carried out in the absence of any noticeable quantities of water. However the starting material may also contain methanol, e.g. up to 25% or minor quantities e.g. not more than 5% of water based on the total weight of water+methyl acetate+methanol. In that case the process yields not only acetic anhydride, but also acetic acid in a quantity approximately equivalent to the quantity of methanol or water employed. The acetic acid can be separated from the main product, acetic anhydride, e.g. by distillation.

In GB 2033385 A (Halcon Research and Development Corporation) is described a process in which a substantially completely anhydrous methyl acetate is prepared from a wet methyl acetate containing significant amounts of water by bringing the wet methyl acetate into contact with acetic anhydride. It is stated that the process is of particular effectiveness and suitability for the dehyration of methyl acetate to be used as feed to a carbonylation reaction where a substantially anhydrous methyl acetate is desired, e.g. for the preparation of acetic anhydride as described in, inter alia, GB 1468940 referred to above. The specification also discloses in claim 3 a process of producing methyl acetate by the esterification of acetic acid with methanol in an esterification zone and carbonylating the resultant wet methyl acetate, after dehydration, in a carbonylation zone to form acetic anhydride, and including the steps of reacting said wet methyl acetate with acetic anhydride in an amount at least substantially stoichiometric equivalent to the water present in said wet methyl acetate in a dehydration zone to produce substantially anhydrous methyl acetate and acetic acid, feeding said substantially anhydrous methyl acetate and said acetic acid to said carbonylation zone, recovering acetic anhydride and acetic acid from said carbonylation zone, separating said acetic anhydride and said acetic acid, directing a portion of said acetic anhydride to said dehydration zone, said portion being said amount at least substantially stoichiometrically equivalent to the water present in said methyl acetate fed to said dehydration zone, and feeding said acetic acid separated from said acetic anhydride to said esterification zone for reaction with methanol to produce said wet methyl acetate.

It is an object of the present invention to provide an improved process for the production of acetic anhydride, with or without the net co-production of acetic acid, from methanol and carbon monoxide as the essential feedstocks in an integrated series of esterification, carbonylation and separation steps, and in which the

product of the esterification reaction containing methyl acetate and water is not subjected to extensive purification or dehydration before feeding to the carbonylation stage.

According to the present invention the process for the production of acetic anhydride with or without the net co-production of acetic acid from methanol and carbon monoxide in a series of esterification, carbonylation and separation steps comprises:

1) reacting methanol with recycle acetic acid in an esterification step to form an esterification product containing predominantly methyl acetate, water and optionally unreacted methanol,

2) removing part of the water from the esterification product,

3) reacting the esterification product still containing water with carbon monoxide in a carbonylation step in the presence as catalyst of free or combined metallic carbonylation catalyst and as promoter of free or combined halogen to form a carbonylation product containing acetic acid and acetic anhydride,

4) separating the carbonylation product by fractional distillation into a low boiling fraction containing carbonylation feed and volatile carbonylation promoter components, acetic acid and acetic anhydride fractions, and a higher boiling fraction containing carbonylation catalyst components,

5) recycling the low boiling fraction containing carbonylation feed and carbonylation promoter components and the higher boiling fraction containing carbonylation catalyst components to the carbonylation step and,

6) recycling at least part of the acetic acid fraction to the esterification step.

With regard to the individual steps, in the esterification step (1) methanol is reacted with recycle acetic acid to form esterification product containing methyl acetate, water and unreacted methanol. In a preferred embodiment recycle acetic acid forms substantially the entire acetic acid in the feed to the esterification. It is an advantage of the present invention that neither the methanol nor the recycle acetic acid need be essentially pure. Thus the methanol may be contaminated with water, for example, and the recycle acetic acid may contain, for example, water, methyl acetate, acetic anhydride, and carbonylation promoter components, such as alkyl halides, e.g. methyl iodide. Though the esterification step may, if desired, be effected in the absence of a catalyst, it is preferred to employ an esterification catalyst, which may be any of the esterification catalysts conventionally employed. Suitable esterification catalysts include mineral acids such as sulphuric acid and organic acids such as toluene-para-sulphonic acid. Alternatively, other esterification catalysts, such as acid ion-exchange resins, may be employed if so desired. The esterification catalyst may suitably be present in an amount from 0.1 to 10%, preferably from 2 to 6% by weight of the reaction mixture.

The esterification step may be effected in a variety of ways. Thus a feed mixture comprising excess methanol and recycle acetic acid may be fed continuously to an esterifier containing aqueous acetic acid and a strong acid catalyst under reflux conditions. A mixture of methyl acetate, water and excess methanol may then be taken overhead as a distillate product. A typical ester distillate product obtained from 2:1 molar ratio feed of methanol:acetic acid comprises 57.5% w/w methyl acetate, 27.9% w/w methanol and 13.8% w/w water. Some of the water may be removed from the product by azeotropic drying and methanol may be removed for recycle, if desired, by hydroselection. Additional distillation steps may be employed to recover methanol from the hydroselection base product and methyl acetate from the decanter water phase/entrainer for recycle. Alternatively, the process described in our published European Application No. 0060717 (BP Case No. 5076) may suitably be employed. In this process methanol is reacted at elevated temperature with acetic acid in the presence of an esterification catalyst and an entrainer which is sparingly soluble in water and which forms a minimum boiling point azeotrope therewith to form a product containing methyl acetate, water and entrainer, an overhead fraction comprising methyl acetate, entrainer and water is distilled from the product mixture in a first column, water is separated from the methyl acetate and entrainer and thereafter methyl acetate is recovered from the entrainer by distillation in a second column. Preferably the process described in our published European application No. 00607196 (BP Case No. 5124) is employed. In this process methanol is reacted at elevated temperature with acetic acid in the presence of an esterification catalyst and an entrainer which is sparingly soluble in water and which forms a minimum boiling point azeotrope therewith to form methyl acetate and water and in a distillation column methyl acetate is recovered as an overhead fraction and from an intermediate point in the column there is removed a liquid sidestream fraction comprising water and entrainer. Entrainers which may be used in the processes of the above referred to European applications Nos. 0060717 and 0060719 include hydrocarbons, ethers, esters and ketones, of which butyl acetate is preferred. The processes are more fully described in the aforesaid UK specifications, the disclosures of which are incorporated herein by reference. Another mode for effecting the esterification step is by counter current esterification. In this mode of operation a single distillation column is employed. To this column acetic acid is fed near the top, methanol is fed near the bottom, a small water stream is added to the reflux to enhance the methanol/methyl acetate separation if required, methyl acetate is removed as head product and water as base product. The column may either be packed with an acid ion-exchange

resin catalyst or a liquid acid catalyst, such as for example sulphuric acid, may be fed to the column.

In step (2), part of the water is removed from the esterification product. In a preferred embodiment the removal of the water from the esterification is effected by removing the water from the reactor vessel in which the esterification takes place.

The reactions which are believed to take place are as follows:

(1) Esterification

$$2CH_3OH+2CH_3COOH \rightarrow 2CH_3COOCH_3+2H_2O$$

(2) Carbonylation

$$2CH_3COOCH_3+2CO \rightarrow 2(CH_3CO)_2O$$

(3) Hydration

$$(CH_3CO)_2O+H_2O \rightarrow 2CH_3COOH$$

The overall equation is therefore:

$$2CH_3OH+2CO \rightarrow (CH_3CO)_2O+H_2O$$

It will be seen from the last equation that, provided some water is removed, the acetic anhydride will be produced.

The greater the proportion of water removed then the higher will be the yield of acetic anhydride rather than acetic acid. Since the object of the present invention is to coproduce acetic acid and acetic anhydride it is important to remove some, but not all, of the water. Preferably water is removed to reduce the water content to below 14% w/w or the equivalent amount of water and methanol for example to below 12% w/w based on the weight of ester+water+alcohol if present. Preferably however at least 6% of water (as water) is present more preferably at least 7% w/w based on the amount of ester+water+alcohol.

In step (3), the esterification product containing predominantly methyl acetate, optionally unreacted methanol, and still containing water is reacted with carbon monoxide in a carbonylation step in the presence as catalyst of free or combined metallic carbonylation catalyst and as promoter free or combined halogen to form a carbonylation product containing acetic acid and acetic anhydride. Any of the known metallic carbonylation catalysts may be employed. Suitable metals include the metals of Group VIII of the Periodic Table of the elements, of which the noble metals iridium, osmium, platinum, palladium, rhodium and ruthenium are preferred. Particularly preferred is rhodium. It is preferred to employ the metal in the form of a soluble compound such as a salt, e.g. a rhodium halide, or a complex of the metal, e.g. a carbonyl complex. As promoter there is used a halogen in free or combined form. Suitable forms include elementary halogen, a hydrogen halide, an inorganic salt, such as for example sodium halide, potassium halide or cobalt halide and quaternary ammonium or phosphonium halide, such as for example tetramethylammonium halide. Particularly preferred as promoters are organohalides, such as alkyl, e.g. methyl iodide, or aryl halides. Methyl iodide is particularly preferred. Suitable reactants and conditions of operation are more fully described in the aforesaid UK Patents Nos. 1468940 and 1,523,346. It is particularly preferred to employ co-promoters. Suitable co-promoters include the metals in free or combined form such, as zirconium and those described in the aforesaid UK Patent No. 1468940, e.g. lithium, magnesium, calcium, titanium, chromium, iron, nickel and aluminium, alkyl or aryl phosphines and organic nitrogen compounds as described in the aforesaid UK patent No. 15233346 and either heterocyclic aromatic compounds in which at least one heteroatom is a quaternary nitrogen atom as described in European Patent No. 8396 or the precursors of heterocyclic aromatic compounds in which at least one heteroatom is a quaternary-nitrogen atom, such as for example N-methyl imidazole. Other suitable promoter systems are described for example in UK Patent No. 1538783 and UK published applications Nos. 2059794, 2067557 and European published application No. 26280.

In one embodiment the carbonylation product from step (3) is separated in a first separation step by fractional distillation into an overhead fraction containing carbonylation feed and carbonylation promoter components, an intermediate fraction containing acetic acid and acetic anhydride, and a lower fraction containing carbonylation catalyst components, the overhead fraction and the lower fraction are recycled to the carbonylation step, the intermediate fraction is separated in a second separation step by fractional distillation into an acetic acid product fraction and an acetic anhydride product fraction, and part or all of the acetic acid product fraction is recycled to the esterification step.

In step (4), the carbonylation product is separated by fractional distillation into a low boiling fraction containing carbonylation feed and carbonylation promoter components, acetic acid and acetic anhydride fractions, and a higher boiling fraction containing carbonylation catalyst components.

In a preferred embodiment of steps (4), (5) and (6) the carbonylation product is separated in a first separation step by fractional distillation into an overhead fraction containing carbonylation feed and carbonylation promoter components, an intermediate fraction containing acetic acid and acetic anhydride, and a lower fraction containing carbonylation catalyst components, the overhead fraction and the lower fraction are recycled to the carbonylation step, the intermediate fraction is separated in a second separation step by fractional distillation into an acetic acid product fraction and an acetic anhydride product fraction, and at least part of the acetic acid product fraction is recycled to the esterification step.

Step (4) may suitably be effected by passing the

carbonylation product to a flash zone from which there is recovered a liquid product which is predominantly acetic acid and anhydride but also contains the metallic catalyst components and any co-promoter present in the product and a volatile product containing acetic acid, acetic anhydride and volatile carbonylation promoter and feed components, e.g. alkyl halides such as methyl iodide and methyl acetate. Optionally, a partial pressure of carbon monoxide and/or hydrogen may be applied in the flash zone to assist in maintaining catalyst activity. Further details may be found in UK patent application publication No. 2037276A which is hereby incorporated by reference. The volatile fraction may suitably be fed to a distillation column wherein volatile carbonylation promoter and feed components are removed overhead optionally together with some acetic acid and anhydride, and recycled to the carbonylation zone, a liquid sidestream comprising acetic acid and acetic anhydride is taken from an intermediate point and a liquid residue containing involatile carbonylation catalyst components carried over from the flash stage is removed from the base for recycle to the carbonylation zone with the liquid product from the flash zone. If the catalyst carry over from the flash stage is acceptable, the acetic acid/anhydride fraction may be taken from the base of the distillation column containing less low boiling impurities. The side or base stream containing acetic acid and acetic anhydride is then fed to a further distillation column from which pure acetic anhydride is removed as base product, and impure acetic acid is removed as overhead product. The impure acetic acid overhead fraction, which may contain some acetic anhydride and small amounts of methyl acetate and methyl iodide depending on whether the acetic acid/anhydride stream was taken as a side stream or a base product from the previous distillation column is then recycled directly to the esterification step. A part of this acetic acid stream may if desired be removed and purified in a further column to produce pure acetic acid.

The process of the present invention is further illustrated with reference to the accompanying drawing which is a simplified flow diagram of a process for the manufacture of acetic anhydride and acetic acid from methanol and carbon monoxide in a series of integrated esterification, carbonylation, and separation steps.

In this process the esterification step is carried out continuously in the kettle of a single distillation column 1, with *n*-butyl acetate as internal entrainer and side decantation to remove water by line 4. Internal entrainer is recycled to the column. Recycle acetic acid is fed to the kettle by line 2, together with an at least equimolar quantity of methanol by line 3, and a mixture of methyl acetate with some water and unreacted methanol is removed as head product by line 5 and passed directly to the carbonylation reactor 6. In the carbonylation reactor 6 the esterification product is reacted with carbon monoxide, fed to the reactor by line 7, in the presence of a rhodium carbonylation catalyst recycled by line 13, a promoter of methyl iodide recycled by line 12, and a co-promoter of N-methyl imidazole recycled as quaternary ammonium salt by line 13. The initial catalyst components are charged by line 8, which is also used for any subsequent make-up. The product of the carbonylation reaction consisting of predominantly acetic anhydride, acetic acid, unreacted methyl acetate and some methyl iodide is passed by line 10 to the separation zone 11 in which it is separated into a low boiling overhead fraction containing carbonylation feed and volatile promoter components which is recycled by line 12 to the carbonylation reactor 6, a high boiling base product containing carbonylation catalyst components which is recycled by line 13 to the carbonylation reactor 6, and a mixed acetic acid/acetic anhydride fraction which is withdrawn as a liquid sidestream by line 14 and passed to the separation zone 15. In zone 15 the acid/anhydride product is separated by fractional distillation into an impure acetic acid overhead fraction, which is recycled, by line 16 to the esterification reactor 1, and an acetic anhydride product fraction which is withdrawn as a base product by line 17. The net acetic acid product from line 16 may be further purified is desired.

Example 1: Parts=weight or weight per hour

In this process the esterification step is carried out continuously in the kettle of a 31 plate Oldershaw column 1. Tray 16 comprises a chimney tray with liquid sidedraw. 3130 parts of methanol are fed to the kettle which contains a refluxing mixture comprising 520 parts of acetic acid, 235 parts of n-butyl acetate as internal entrainer, 10 parts of water and 40 parts of methane sulphonic acid catalyst. The temperature at tray 15 is maintained at 80 to 90°C. The sidestream from the chimney tray is cooled and separated into two phases in a decanter. The lower oil phase containing the internal entrainer is returned to tray 15. 1925 parts of aqueous phase is discharged by line 4. Recycle acetic acid (5450 parts) is fed to the kettle by line 2. A mixture of 6500 parts methyl acetate, 200 parts water and 150 parts methanol is removed as a head product by the line 5, after maintaining a 5:2 reflux to the column. The product in line 5 is made up with 220 parts of methyl acetate and 70 parts of methanol corresponding to material which could be recovered in a simple stripping stage from the waste water stream in line 4, together with 550 parts of water to give a total of 9.8% water in the feed to the carbonylation reactor 6. The carbonylation reactor 6 comprises a stirred autoclave in corrosion resistant material in which the methyl acetate is reacted with carbon monoxide sparged by line 7. The reactor contains 4290 parts of a mixture containing 14 parts of rhodium expressed as rhodium diacetate, together with 40 parts of zirconyl diacetate and 215 parts of N,N'-dimethyl imidazolinium iodide. The balance

comprises methyl acetate, acetic acid, acetic anhydride, and acetophenone as high boiling solvent. The carbonylation reactor 6 is maintained at a temperature of 183°C and a total pressure of 30 bar absolute. The initial catalyst components are charged by line 8, which is also used for any subsequent make-up. The product of the carbonylation reaction consisting of predominantly acetic anhydride, acetic acid, methyl iodide, solvent and unreacted methyl acetate is passed by line 10 to the separation zone 11 which is maintained at a pressure of 3 bar absolute. A high boiling base product containing all of the rhodium catalyst and zirconium and quaternary ammonium salt promoters is recycled by line 13 to the carbonylation reactor 6 together with the acetophenone and some acetic acid and acetic anhydride. A low boiling overhead fraction containing most of the unreacted methyl acetate carbonylation feed and volatile unquaternarised methyl iodide promoter is recycled by line 12 to the carbonylation reactor 6. 10,490 parts of a mixture of acetic acid and acetic anhydride vapourised in the flash stage of separation zone 11 at a temperature of ca 135°C is recoverable as an intermedaite fraction by a liquid sidestream and passed by line 14 to the separation zone 15. This comprises a 40 plate Oldershaw column with the feedpoint at plate 15 maintained at a temperature of 126°C to give a measured kettle temperature of 143°C and a heads temperature of 118°C 5030 parts of a base product are recovered by line 17 containing 98.5% of acetic anhydride. With a column reflux of 2:1 an overhead product of 5460 parts of acetic acid is obtained by line 16, containing trace levels of methyl acetate and methyl iodide. All the acetic acid is recycled to the esterification column 1 by line 2.

Example 2

The process is repeated as Example 1, save that the water in line 5 to the carbonylation stage is made up to 955 parts corresponding to 12.1% of the carbonylation feed. In this case 10,640 parts of mixed acetic acid and acetic anhydride is recovered from the separation zone 11 and fed by line 14 to separation zone 15. 3845 parts of acetic anhydride are recovered as a product by line 17 and a net acetic acid product of 1330 parts recovered by line 16 for use or further purification if required.

The above described process has the following advantages:

The acetic acid for the esterification step (or at least a significant portion thereof) is passed directly from the separation stage (in which it is separated from the acetic anhydride) without treatment. This means the esterification feed is not dependent on recovered acetic acid from subsequent operations involving the use of acetic anhydride.

The process offers flexibility in acid/anhydride product ratios without the need for a separate facility to hydrolyse anhydride to acid.

The esterification product is passed directly from the esterification reactor (from which water is removed) to the carbonylation reactor without any treatment such as purification or drying.

## Claims

1. A process for the production of acetic anhydride with or without the net co-production of acetic acid from methanol and carbon monoxide in a series of esterification, carbonylation and separation steps comprising:

1) reacting methanol with recycle acetic acid in an esterification step to form an esterification product containing predominantly methyl acetate, water and optionally unreacted methanol,

2) removing part of the water from the esterification product,

3) reacting the esterification product still containing water with carbon monoxide in a carbonylation step in the presence as catalyst of free or combined metallic carbonylation catalyst and as promoter of free or combined halogen to form a carbonylation product containing acetic acid and acetic anhydride,

4) separating the carbonylation product by fractional distillation into a low boiling fraction containing carbonylation feed and volatile carbonylation promoter components, acetic acid and acetic anhydride fractions, and a higher boiling fraction containing carbonylation catalyst components,

5) recycling the low boiling fraction containing carbonylation feed and carbonylation promoter components and the higher boiling fraction containing carbonylation catalyst components to the carbonylation step and,

6) recycling at least part of the acetic acid fraction to the esterification step.

2. A process as claimed in Claim 1 wherein the carbonylation product is separated in a first separation step by fractional distillation into an overhead fraction containing carbonylation feed and carbonylation promoter components, an intermediate fraction containing acetic acid and acetic anhydride, and a lower fraction containing carbonylation catalyst components, the overhead fraction and the lower fraction are recycled to the carbonylation step, the intermediate fraction is separated in a second separation step by fractional distillation into an acetic acid product fraction and an acetic anhydride product fraction, and part or all of the acetic acid product fraction is recycled to the esterification step.

3. A process as claimed in Claim 1 or Claim 2 wherein recycle acetic acid forms at least 50% of the acetic acid in the feed to the esterification.

4. A process as claimed in claim 3 wherein the amount of water in the feed to the carbonylation step is at least 6% w/w based on the weight of ester and methyl alcohol if present so that sufficient acetic acid is produced whereby the recycle acid forms at least 50% of the acetic acid in the feed to the esterification.

5. A process as claimed in claim 4 whereby the

amount of water and alcohol (if present) is such that more acetic acid is produced in the carbonylation step than is required for the acetic acid in the feed to the esterification so that the process produces a net amount of acetic acid.

6. A process as claimed in any one of the preceding claims wherein the esterification product, after the removal of water in step (2) is passed directly to the carbonylation step (3).

7. A process as claimed in any one of the preceding claims wherein the recycle acetic acid to the esterification stage is passed directly from the carbonylation product separation stage in which the acetic acid and acetic anhydride are separated from each other.

**Patentansprüche**

1. Verfahren zur Herstellung von Essigsäureanhydrid mit oder ohne Coproduktion von Essigsäure aus Methanol und Kohlenmonoxid in einer Serie von Veresterungs-, Carbonylierungs- und Trennungsstufen, im wesentlichen bestehend aus:

1) Umsetzung von Methanol mit rückgeführter Essigsäure in einer Veresterungsstufe zur Bildung eines Veresterungsproduktes enthaltend vorwiegend Methylacetat, Wasser und gegebenenfalls nicht-umgesetztes Methanol,

2) Entfernung eines Teils des Wassers aus dem Veresterungsprodukt,

3) Umsetzung des Veresterungsproduktes, welches noch Wasser enthält, mit Kohlenmonoxid in einer Carbonylierungsstufe in Anwesenheit von freiem oder kombinierten metallischen Carbonylierungskatalysator und freiem oder kombinierten Halogen als Promotor zur Bildung eines Carbonylierungsproduktes enthaltend Essigsäure und Essigsäureanhydrid,

4) Trennung des Carbonylierungsproduktes durch fraktionierte Destillation in eine niedrigsiedende Fraktion enthaltend Carbonylierungs-Ausgangsmaterial und flüchtige Carbonlierungs-Promotor-Komponenten, Essigsäure und Essigsäureanhydrid-Fraktionen, und eine höhersiedende Fraktion enthaltend Carbonylierungs-Katalysator-Komponenten,

5) Rückführung der niedrigsiedenden Fraktion enthaltend Carbonylierungs-Ausgangsmaterial und Carbonylierungs-Promotor-Komponente und den höher siedenden Fraktion enthaltend Carbonylierungs-Katalysator-Komponenten in die Carbonylierungsstufe und

6) Rückführung von zumindest einem Teil der Essigsäurefraktion in die Veresterungsstufe.

2. Verfahren gemäß Anspruch 1, worin das Carbonylierungsprodukt in einer ersten Trennungsstufe durch fraktionierte Destillation getrennt wird in eine Kopffraktion enthaltend Carbonylierungs-Ausgangsmaterial und Carbonylierungs-Promotor-Komponenten, eine mittlere Fraktion enthaltend Essigsäure und Essigsäureanhydrid und eine niedere Fraktion enthaltend Carbonylierungs-Katalysator-Komponente, wobei die Kopffraktion und die niedere Fraktion rückgeführt werden in die Carbonylierungsstufe, die Mittelfraktion getrennt wird in einer zweiten Trennstufe durch fraktionierte Destillation in eine Essigsäure-Produkt-Fraktion und eine Essigsäureanhydrid-Produkt-Fraktion und ein Teil oder die gesamte Essigsäure-Produkt-Fraktion in die Veresterungsstufe rückgeführt wird.

3. Verfahren gemäß Anspruch 1 oder 2, worin die rückgeführte Essigsäure mindestens 50% der Essigsäure in dem Ausgangsmaterial zu der Veresterung ausmacht.

4. Verfahren gemäß Anspruch 3, worin die Menge an Wasser in dem Ausgangsmaterial zu der Carbonylierungsstufe mindestens 6 Gew./Gew.-% bezogen auf das Gewicht von Ester und Methylalkohol, sofern vorhanden, ausmacht, so daß genügend Essigsäure produziert wird, wobei die rückgeführte Säure mindestens 50% der Essigsäure in dem Ausgangsmaterial der Veresterung ausmacht.

5. Verfahren gemäß Anspruch 4, wobei die Menge an Wasser und Alkohol (sofern vorhanden) derart ist, daß mehr Essigsäure produziert wird in der Carbonylierungsstufe als benötigt wird für die Essigsäure in dem Ausgangsmaterial zur Veresterung, so daß das Verfahren einen Nettosaldo an Essigsäure liefert.

6. Verfahren gemäß einem der vorgehenden Ansprüche, worin das Veresterungsprodukt nach der Entfernung von Wasser in Stufe 2 direkt in die Carbonylierungsstufe 3 geführt wird.

7. Verfahren gemäß einem der vorgehenden Ansprüche, worin die Essigsäure direkt in die Veresterungsstufe zurückgeführt wird aus der Trennstufe des Carbonylierungsproduktes, in welcher die Essigsäure und das Essigsäureanhydrid voneinander getrennt werden.

**Revendications**

1. Procédé de fabrication d'anhydride acétique, avec ou sans production résultante simultanée d'acide acétique, à partir de méthanol et d'oxyde de carbone, dans une série d'étapes d'estérification, de carbonylation et de séparation, comprenant:

1) la réaction du méthanol avec l'acide acétique recyclé, dans une étape d'estérification destinée à former un produit d'estérification contenant essentiellement de l'acétate de méthyle, de l'eau et éventuellement du méthanol qui n'as pas réagi,

2) l'extraction d'une partie de l'eau du produit d'estérification,

3) la réaction du produit d'estérification contenant encore de l'eau avec de l'oxyde de carbone dans une étape de carbonylation, en présence, sous forme d'un catalyseur, d'un catalyseur métallique libre ou combiné de carbonylation et, sous forme d'un activateur, d'un halogène libre ou combiné afin qu'un produit de carbonylation contenant de l'acide acétique et de l'anhydride acétique se forme,

4) la séparation du produit de carbonylation par distillation fractionnée, en une fraction à faible

température d'ébullition contenant des ingrédients de la charge de carbonylation et de l'activateur volatil de carbonylation, des fractions d'acide acétique et d'anhydride acétique, et une fraction à température élevée d'ébullition contenant des ingrédients du catalyseur de carbonylation,

5) le recyclage de la fraction à faible température d'ébullition, contenant des ingrédients de la charge de carbonylation et de l'activateur de carbonylation et de la fraction à température élevée d'ébullition contenant des ingrédients du catalyseur de carbonylation, vers l'étape de carbonylation, et

6) le recyclage d'une partie au moins de la fraction d'acide acétique vers l'étape d'estérification.

2. Procédé selon la revendication 1, dans lequel le produit de carbonylation est séparé dans une première étape de séparation, par distillation fractionnée, en une fraction de tête contenant les ingrédients de la charge de carbonylation et de l'activateur de carbonylation, une fraction intermédiaire contenant de l'acide acétique et de l'anhydride acétique, et une fraction inférieure contenant des ingrédients du catalyseur de carbonylation, la fraction de tête et la fraction inférieure étant recyclées vers l'étape de carbonylation, la fraction intermédiaire étant séparée dans une seconde étape de séparation par distillation fractionnée, en une fraction constituant de l'acide acétique produit et une fraction constituant de l'anhydride acétique produit, et une partie ou la totalité de la fraction d'acide acétique produit est recyclée vers l'étape d'estérification.

3. Procédé selon l'une des revendications 1 et 2, caractérisé en ce que l'acide acétique recyclé forme au moins 50% de l'acide acétique de la charge d'estérification.

4. Procédé selon la revendication 3, dans lequel la quantité d'eau présente dans la charge de l'étape de carbonylation est au moins égale à 6% en poids/poids par rapport au poids de l'ester et de l'alcool méthylique le cas échéant afin qu'une quantité suffisante d'acide acétique soit produite, si bien que l'acide recyclé forme au moins 50% de l'acide acétique de la charge d'estérification.

5. Procédé selon la revendication 4, dans lequel la quantité d'eau et d'alcool (le cas échéant) est telle que la quantité d'acide acétique produite dans l'étape de carbonylation est plus grande que la quantité nécessaire à la formation de l'acide acétique de la charge d'estérification si bien que le procédé produit une quantité nette d'acide acétique.

6. Procédé selon l'une quelconque des revendications précédentes, dans lequel le produit d'estérification, après l'enlèvement d'eau dans l'étape (2) est transmis directement à l'étape de carbonylation (3).

7. Procédé selon l'une quelconque des revendications précédentes, dans lequel l'acide acétique recyclé vers l'étape d'estérification est transmis directement de l'étage de séparation du produit de carbonylation dans lequel l'acide acétique et l'anhydride acétique sont séparés l'un de l'autre.

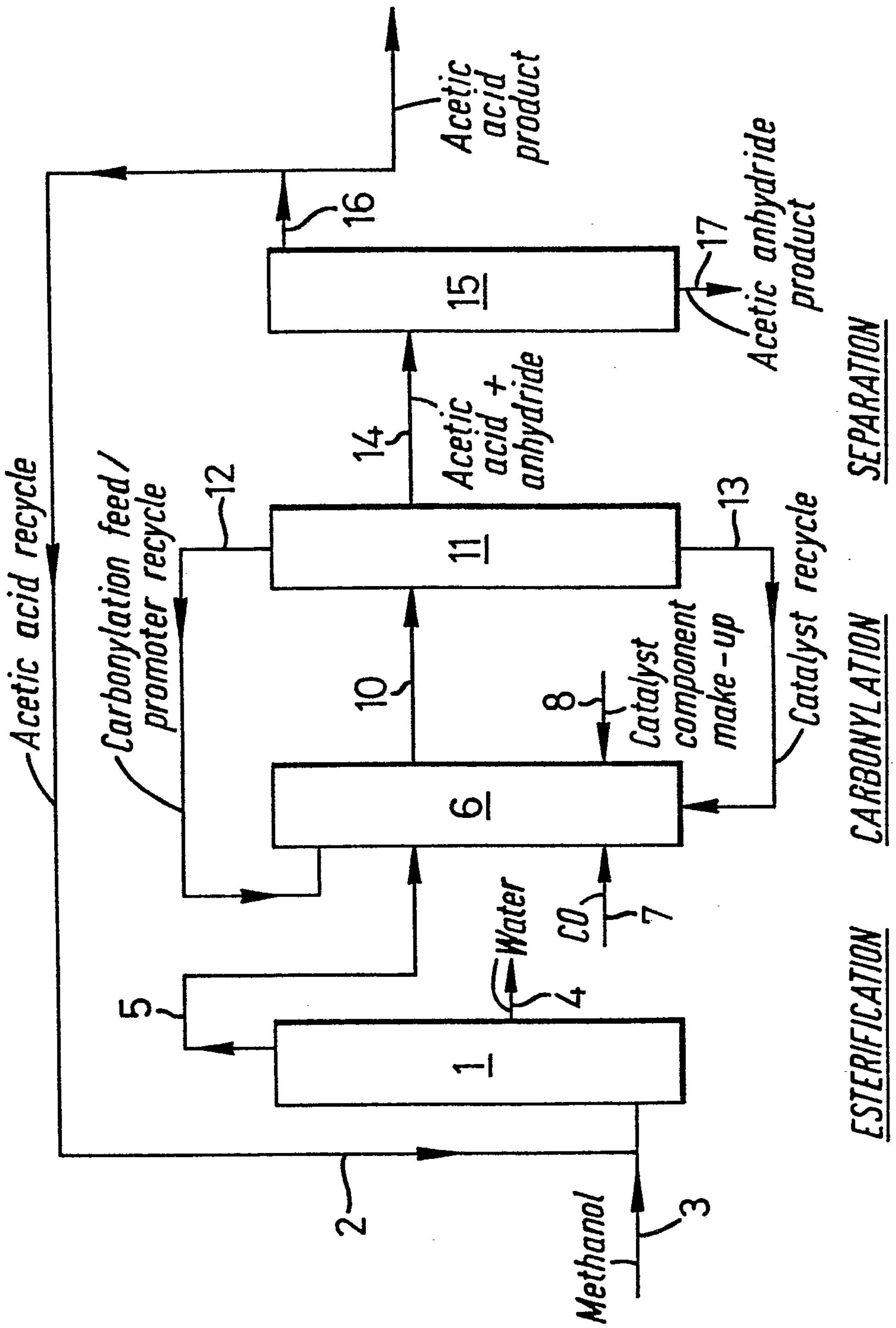

Acetic acid product
16
Acetic acid recycle
15
17
Acetic anhydride product
SEPARATION
Carbonylation feed / promoter recycle
12
11
14
Acetic acid + anhydride
13
Catalyst recycle
CARBONYLATION
10
8
Catalyst component make-up
6
Water
4
CO
7
5
1
ESTERIFICATION
2
Methanol
3